# EUROPEAN PATENT SPECIFICATION

(11) **EP 1 791 474 B1**
(45) Date of publication and mention of the grant of the patent: **01.07.2015**
(21) Application number: 05778002.5
(22) Date of filing: 09.09.2005
(51) Int. Cl.: A61B 17/04

(54) **BUTTON ANCHOR SYSTEM FOR MOVING TISSUE**
KNOPFANKERSYSTEM ZUR VERSCHIEBUNG VON GEWEBE
SYSTEME DE BOUTON D'ANCRAGE POUR DEPLACEMENT DE TISSU

(30) Priority: 09.09.2004 US 608686 P
(43) Date of publication of application: 06.06.2007
(73) Proprietor: Canica Design Inc., Almonte, Ontario K0A-1A0 (CA)
(72) Inventor: HENDERSON, James, Gatineau PQ J9H 5E1 (CA); O'MALLEY, Michael, T., Appleton, Ontario K0A 1A0 (CA); MAXWELL, Timothy, Carp, Ontario K0A 1L0 (CA); REITSMA, Bert, Manotick, Ontario K4M 1B2 (CA)
(74) Representative: Boff, James Charles
(86) International application number: PCT/IB2005/002679
(87) International publication number: WO 2006/027678

(56) References cited:
- WO-A1-92/15251
- US-A- 1 852 098
- US-A- 2 075 508
- US-A- 3 664 345
- US-A- 5 041 129
- US-A- 5 269 809
- US-A1- 2003 092 969

## Description

### Field of the Invention

This invention relates generally to a system for moving or for moving and stretching human or animal plastic tissue that exerts a relatively constant tension over a given distance and that is readily adjustable, and more specifically to an anchor for use with such systems.

### Background

In general, surgery and surgical treatment involve one or both of tissue separation and tissue joining. In surgery, medical treatment, and medical research, it is desirable to retract tissue, stabilize tissue, and present tissue in a variety of specific orientations to provide access to the area under investigation or repair, ideally in a method that creates minimal trauma beyond what is necessary for exposure and visualization of the operative area. Ultimately, the procedures should allow for immediate, or primary, closure of the wound. Unfortunately, the latter option is not always available in surgical or trauma wound scenarios.

Moving tissue presents unique challenges, as tissues often resist joining, or closure, depending on the nature of the tissue structure, the circumstances of the tissue separation, and general patient health. Complications related to wound closure and healing generally result from major forces, minor forces and/or compromised healing responses. Major forces are retractive forces created beyond the viscoelastic properties of the tissue, and may be created by: (1) increased internal volume, such as in the case of obesity, which elevates containment forces on the skin system; (2) changes in aspect ratio, such as increased abdominal circumference created in prone, non-ambulatory patients due to muscular atrophy; (3) respiratory muscular activity; (4) muscular response; (5) loss of fascia structure; (6) muscular-skeletal deformation; (7) fleshy appendages; (8) tumors; and (9) severe burns.

Minor forces are internal forces created by the viscoelastic properties of the tissue, which can cause the skin to retract. Elastic tissues, such as skin, comprised mostly of extracellular matrix (ECM) components along with cells, return to a minimum elastic, or relaxed, state when released from tension. In this relaxed state, tissue tensions are minimized and balanced. Skin tissue in this minimum elastic state will remain relaxed, demonstrating behavior similar to a non-elastic material. The force required to elongate the tissue in this state often approaches a force that will rupture or sheer structural connective elements, causing localized failures or tears. Soft tissue in this minimum elastic state provides minimum surface coverage and has the highest reluctance to stretch. It is known that a gentle but constant force below the sheer force threshold applied to tissue in combination with adequate hydration will, over time, restore certain tissues to near-original or original elastic state. Additionally, this force can be applied to stretch tissue past the point of equilibrium (normal elastic range) to the maximum elastic range and create the thinnest possible configuration, covering the maximum surface area. If tensions in the tissue do not exceed the point at which the connective structural elements are compromised, the tissue remains at the maximum elastic state as healthy tissue, and normal biological processes will allow cell regeneration and associated ECM production to restore normal skin thickness and tension, which is described below as biological creep.

Plastic tissues, such as skin and muscle, possess certain viscous and elastic rheological properties, and are therefore viscoelastic. Certain plastic tissues are able to increase surface area over time, which can be termed "creep." "Mechanical creep" is the elongation of skin with a constant load over time beyond intrinsic extensibility, while "biological creep" refers to the generation of new tissue due to a chronic stretching force. A constant and unrelenting force applied to a body tissue, such as skin or muscle, may result in both mechanical and biological creep. Mechanical creep restores the tension originally present but lost in the skin across the incision or wound by retensioning skin, thereby increasing skin coverage. Biological creep occurs more slowly and involves the creation of new tissue. Tissue expansion has long been part of the art of plastic surgery, traditionally accomplished with balloon-type tissue expanders embedded under the skin and externally inflated and increased over time to create expanded pockets of skin for procedures such as breast reconstruction after radical mastectomies, and stretching healthy tissue prior to plastic surgery for the creation of flaps for soft tissue closure.

Finally, compromised healing responses may complicate wound closure or healing. A surgical or other incision becomes a complicated wound as soon as it falls behind normal healing progression. Wound management, including treatment and care of large skin defects and severely retracted incisions, is an area of increasing importance to the health care community. An aging population and an increase in diseases related to obesity and inactivity have increased the occurrence of complicated wounds and placed an increased burden on health care resources. Factors contributing to compromised wound healing include patient age, weight, nutritional status, dehydration, blood supply to the wound site, immune response, allergies to closure materials, chronic disease, debilitating injuries, localized or systemic infection, diabetes, and the use of immunosuppressive, corticosteroid or antineoplastic drugs, hormones, or radiation therapy. Complicated wounds include, but are not limited to: surgical wounds, diabetic ulcers and other chronic ulcers; venous stastis ulcers; decubitis or pressure sores or ulcers; bums; post traumatic lesions, cutaneous gangrene, crush wounds with ischemic necrosis; wounds having exposed plates or bones; keloids; skin lesions; blunt abdominal trauma with perforations; and other acute, subacute or chronic wounds. Treatment and care of these tissue defects is challenging due to difficulties in closure of open wounds.

Two common methods of closure of wounds and skin defects include split thickness skin grafting and gradual closure. A split thickness skin graft involves removing a partial layer of skin from a donor site, usually an upper leg or thigh, and leaving a portion of the dermis at the donor site to regenerate and re-epithelialize. In this manner, a viable skin repair patch can be transferred or grafted to cover a wound area. The graft is often meshed, (which involves cutting the skin in a series of rows of offset longitudinal interdigitating cuts) allowing the graft to stretch to cover two or three times greater an area as well as provide wound drainage while healing. Normal biological function of the skin heals the holes after the graft has been accepted. A meshed graft of this type requires a smaller donor area than a conventional non-meshed or full thickness skin graft. However, these methods do not provide optimal cosmesis or quality of skin cover. Other disadvantages of this method include pain at the donor site, creation of an additional disfiguring wound, and complications associated with incomplete "take" of the graft. In addition, skin grafting often requires immobilization of the limb, which increases the likelihood of contractures. The additional operation and prolongation of hospital stay is an additional economic burden.

Gradual, or progressive, closure is a second method of closure. This technique may involve suturing vessel loops to the wound edge and drawing them together with large sutures in a fashion similar to lacing a shoe. In addition, the wound edges may be progressively approximated with suture or sterile paper tape. The advantages of this gradual, or progressive, technique are numerous: no donor site is required for harvest of a graft, limb mobility is maintained, and superior cosmetic result, more durable skin coverage, better protection from full skin thickness and the maintenance of normal skin sensation may all be achieved.

Existing devices for effecting a gradual closure have many disadvantages. Current methods and devices draw wound edges together using mechanical devices such as screw-actuated systems that require repeated periodic adjustment because a relatively small skin movement substantially eliminates much of the closure force. Widely used existing closure techniques involve use of relatively inelastic materials, such as sutures or surgical tape. Excessive tension may cut the skin or cause necrosis due to point loading of the tissue. Current solutions include suture bolsters, suture bridges, use of staples as anchors at the wound edge, and the use of ligature wire to distribute the load along the wound margins. These approaches all rely on static ribbon or suture material, which must repeatedly be readjusted in order to function effectively, and even with this constant readjustment, maintenance of near constant tension over time is difficult, if not impossible, to achieve. Widely used traditional gradual closure methods rely on static force through fixed distance reduction, and do not provide continuous or dynamic tension.

Many current methods of open wound reduction employ static or non-yielding devices such as sutures or hard approximators, which reduce the distance between the wound margins and rely on the skin's natural elasticity to compensate for movement. One problem with these devices has been that when they are at the point of being most effective, when the skin is at the point of maximum stretch, additional skin tension created through motion, such as breathing or walking, creates stress points where the mechanical fasteners meet the wound margins, causing tearing and wound edge necrosis. This has generally required patients to remain immobile during the course of treatment. Existing systems for treating animals need not consider cosmetic result to such a degree as the healthy patient typically masks the wound site with fur, but cosmesis is a critical criteria in the measurement of a successful result from the system in the human application.

One existing method for effecting closure of a wound utilizes a constant tension, low-grade force to draw wound edges together. One device for practicing this method includes a pair of hooks carried by a pair of sliders that move along a path pulled by a pair of springs. This spring device is enclosed in a plastic housing and is available having various curvatures. The sharp hooks used in this system may damage the skin. The constant force used is a dictated force that is not variable. Other closure devices use elastomeric material, including rubber bands and other types of compressive and non-compressive materials, to approximate wound margins. One kit requires bonding to the skin with an adhesive and also requires periodic adjustment to tighten the straps. Other known closure devices use hooks and elastic loops, which must be replaced with smaller elastic loops to maintain tension, or a motor power source to provide a tightening means. Finally, another current device consists of two surgical needles, two U-shaped lexan polycarbonate arms with hooks on the bottom surface, a threaded tension bar and a polycarbonate ruler. The needles are threaded along the wound margin and each arm is positioned above a needle, with the hooks piercing the skin and engaging the needles. The tension bar is then locked, and tension can be adjusted using the screw.

Existing methods of gradual wound closure fail to provide an effective gradual closure that restores original skin tensions lost across the wound. For example, one system has a single tension of 460 grams. In many instances, such as with the elderly or with compromised skin, this force is too great, resulting in localized failures, tears and necrosis. Many current devices are cumbersome, restrict patient mobility, must be completely removed for wound dressing and cleaning, and are usable in a relatively limited number of situations because of size constraints. Many also require a surgeon for reinstallation after removal for wound dressing. Finally, many current devices cannot readily be used for radial closure of wounds due to their limited ability to pull in a single direction along an overhead beam, thereby restricting their application to parallel pulls along the same axis.

Document US 2003/092969 discloses a system according to the preamble of claim 1.

### Summary of the Invention

This invention provides manipulation and control of tissue positions and tensions on a living person or animal, utilizing both tissue stretch and creep to restore and move tissues. This invention provides devices for moving or for moving and stretching tissue that are simple, easy to use, cost-effective, extremely versatile, self-adjusting and capable of exerting relatively constant force or tension over a variety of distances and at various intersecting angles in wounds having simple or complex geometry.

Components of this invention exert a dynamic force on the tissue, providing and maintaining a maximum safe counter-traction pressure or force across a wound margin or other area. Systems of this invention create controlled constant and unrelenting tension, which can be applied to counteract major or minor retraction forces or to achieve maximum mechanical and biological yields to move or to move and stretch plastic tissue, including closure of large retracted skin defects.

Terms used herein are generally defined and, in some cases, abbreviated, as they are introduced. For convenience, selected terms are also defined here. A force applying component ("fac") generally stores energy in a manner that exerts force and transmits the force. An elastic force applying component ("efac") combines these two functions in a single elastic component. The tissue manipulation system of this invention utilizes facs coupled to force coupling components ("anchors") that couple to tissue the force exerted by the force applying component. The term "elastomer" refers to relatively elastic material, such as silicone, or latex rubber. The term "non-reactive" is used to describe components that are either immunologically inert or hypoallergenic.

Coupling a fac to tissue can occur simply by passing a fac or a portion of a fac such as a suture through a hole created to penetrate tissue. However, such rudimentary coupling works poorly for several reasons, importantly including the extremely poor force distribution across the tissue and the absence of any practical means for adjusting the force exerted by the suture over a period of time.

Anchors of this invention include structures for coupling to force applying components that permit quick, easy attachment and reattachment of various facs, particularly including facs made of silicone, which is extremely difficult to secure. Anchors of this invention provide distribution of force applied and bolster tissue proximate holes through which an fac passes.

This invention provides advances over current methods for moving or moving and stretching plastic tissue through the introduction of gradual but unrelenting tension that is readily adjustable. When tension adjustment is required, it can be accomplished quickly, and the force applying components can include an easily read quantitative visual indicator. Utilizing dynamic force to move and stretch tissue offers the advantage of a relentless countertraction force, while allowing for expansion and contraction of the wound site, which greatly enhances patient mobility and is compliant with respiratory movements.

This invention can be used to apply dynamic force for closure or remodeling of tissue to close dermal wounds, incisions, or defects that may be associated with a variety of conditions, as well as in the stretching of healthy skin in preparation for a skin graft, flap or other remodeling procedure. In one example, this invention includes a system of button anchor assemblies for moving or for moving and stretching plastic tissue, particularly including deep fascia and muscle layers of the abdominal or thoracic cavity wall, in surgical, post surgical, and post traumatic reconstruction where the wound margins are beyond a distance that permits normal re-approximation.

Prior patent applications assigned to Canica Design Inc. describe in detail the use of elastomers and anchors to move and stretch tissue. While the structures disclosed are highly effective, this invention extends the principles disclosed in the earlier patent applications to additionally provide different anchors for the re-approximation of severely retracted abdominal wall and full thickness thoracic wounds where a closure force is required to be applied to the sub-dermal layers. Systems of this invention allow for such a force to be applied and externally controlled during treatment.

The invention is as disclosed in the appended set of claims.

### Brief Description of the Drawings

Figure 1 is a perspective view of a system of this invention for moving tissue.
Figure 2 is a perspective view of a button anchor and anchor tail of the system of Figure 1.
Figure 3 is a top view of the button anchor of Figure 2.
Figure 4 is a front view of the button anchor of Figure 2.
Figure 5 is a top view of the anchoring portion of the button anchor of Figure 2.
Figure 6 is a top view of the anchor pad of the button anchor of Figure 2.
Figure 7 is a perspective view of the anchor pad of the button anchor of Figure 2.
Figure 8 is a top view of the anchor tail of the button anchor of Figure 2.
Figure 9 is an enlarged detail perspective view of a portion of the anchoring portion of the button anchor of Figure 2 showing the anchor tail locking interface.
Figure 10 is a perspective view of installation of part of the system of Figure 1.

### Detailed Description

Anchors of this invention are used to transmit and distribute force to the tissue to be moved or stretched. A force applying component according to this invention may be formed in rods, cords, bands, loops, sheets, nets, wires, strands, cables, tubes or other suitable structure. In one embodiment, the fac is an elastic strand that flattens out at the point of maximum load and becomes load dissipating. In one embodiment, a rod-shaped fac is driven through the tissue using a cannula-like device and is attached at each end to an anchor.

Force applying components ("facs") of this invention may have elastic properties "efacs") and may be made from any suitable elastomeric material, including, without limitation, latex rubber, silicone, natural rubber and materials of similar elasticity, GR-S, neoprene, nitrile-butyl-polysulfide, ethylene-polyurethane, polyurethane, or any other suitable material that exhibits the property of exerting a return force when held in an elongated state at tensions and distances that are useful in the context of this invention. Efacs may provide a dynamic opposing force equal to or greater than the naturally occurring elastomeric traction forces of the tissue. The efacs of this invention generally are not endless loops but rather are lengths of a single strand, sometimes called a "monostrand," and may be either solid or hollow. In some instances, multiple strands or endless loops or bands may be used. Significantly, the efacs used in practicing this invention may be secured to a tissue attachment structure at virtually any point along the efac, providing variable tension within the elastic limits of the elastomer used. Use of a non-reactive fac is generally desirable. Non-reactive facs include components that are either immunologically inert or hypoallergenic, such a elastomers formed from silicone or a hypoallergenic form of latex rubber.

Elastomers having various durometers may be used for the force applying components of this invention. Although other elastomeric materials and sizes of material may be used, polyurethane, thermoplastic (TPE) or rubber elastomer in monofilaments 1mm - 8mm in diameter have been found to be useful in practicing this invention.

In one embodiment, an efac has a 0.318 cm (0.125 inch) diameter with a nominal durometer of 40. Other efacs, such as efacs having a smaller diameter, may also be provided and differentiated one from another based on color. Alternative shapes, sizes and strengths may be appropriate in some situations. An extruded silicone efac may have a durometer of 40 (which allows a 5:1 stretch ratio). A molded silicone efac may have a durometer of 5 (which allows a 12:1 stretch ratio). In one example, a secure mechanical lock may be achieved by restraining the efac within a constricting aperture of a size greater than the tensioned diameter but less than the untensioned diameter, such that the untensioned end of the elastomer acts as a restraint upon the aperture.

Force applying components can include marks indicating tension or stretch. The indicia may be formed from colorant, including any means for providing visual contrast, such as ink, dye, paint, or the like. Force applying components may also be disposable.

As noted above, it is generally desirable to use a non-reactive elastomeric force applying component such as a silicone, but silicone is normally difficult to secure. The viscoplastic properties of low durometer material, such as silicone, fall below the threshold where the material will hold a knot. Adequate constricting force may not be applied upon the material by the material itself to retain it under load because the application of the load reduces the material diameter beyond the minimum compression diameter of the constricting loop. This precludes the use of conventional surgical knot tying techniques because such knots will not hold. An additional complication is the tendency of the material to creep, or slip, when alternative capture methods are used. Thus, it is difficult to secure a silicone efac when a force is applied to the efac without the efac being cut or otherwise caused to fail by the securing structure.

Successful structures for securing a silicone elastomer (or other low durometer material) must clamp the silicone elastomer structure with enough force to hold it in place (avoiding creep) but with sufficiently distributed force that the elastomer is not severed. This invention provides structures that result in sufficient contact between an efac (including a silicone efac) and anchor structure that the two do not slide relative to each other while avoiding cutting or tearing the efac. Such structure can be provided by squeezing the efac between, or forcing it against, planar or relatively large radius arcuate surfaces while avoiding contact between the efac and arrises (intersections of planar surfaces) that might cut the elastomer.

Such a structure can be achieved with opposed planar or arcuate surfaces forming a Vee-shape and oriented so that tension on the efac forced into the gap between the surfaces will cause any reduction in outer diameter of the efac, such as occurs with added load, to result in the efac securing purchase lower in the Vee. In this manner, the efac-to-anchor structure contact is maintained, thereby improving the lock between the elastomer and anchor structure. Similarly, parallel surfaces may be engineered to provide an entrapment force and prescribed release tension for the efac in order to provide a maximum applicable tension and integral safety release.

The opposed surfaces can be provided by a variety of structures, such as arcuate surfaces provided by suitably rigid round wire or rod or by rounded opposed edges of plates of metal, plastic or other suitable material. Such structure can also be provided in other forms. For instance, the opposed surfaces between which the efac is trapped can also be provided by opposed flanges, typically positioned on a post or column and shaped so that the opposed flange surfaces get progressively closer together at points nearer the column. In such a structure, a first one of the opposed surfaces can be planar and can be, for instance, a flat base, provided that the other flange or other efac contact structure provides a surface that gets progressively closer to the first surface as the efac moves in the direction force applied to it during use will cause it to tend to move. For instance, the other flange can present a truncated conical surface.

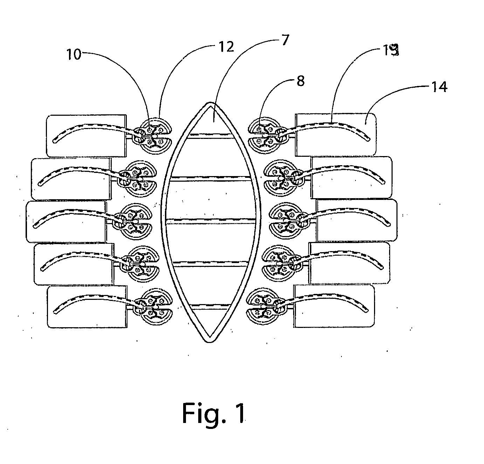

As shown in Figures 1-3, a button anchor 8 of this invention comprises an anchoring portion 10, which rests on an anchor pad 12 and which can optionally engage a load distributing anchor tail 14. This button anchor 8 remains external to the human or animal tissues, and comprises specific features for anchoring a fac traveling across a wound or through tissues that, by its presence and ability to apply a reducing force, provides the specific benefit of moving or moving and stretching tissue to bring reduction or closure of a full thickness wound where the wound margins lie beyond a distance where they can be primarily closed without undue force. In one example, a fac is passed through the skin, engaging or encircling the sub-dermal structures requiring closure, and returned through the skin on the other side of a wound or incision. The button anchors 8 are applied to the ends of the fac, allowing the fac to be tensioned and anchored, thereby applying a sub-dermal reduction force, as illustrated in Figure 1. In an alternative embodiment, button anchors 8 positioned on opposite sides of a wound secure a fac that passes over the wound and that does not penetrate the tissue.

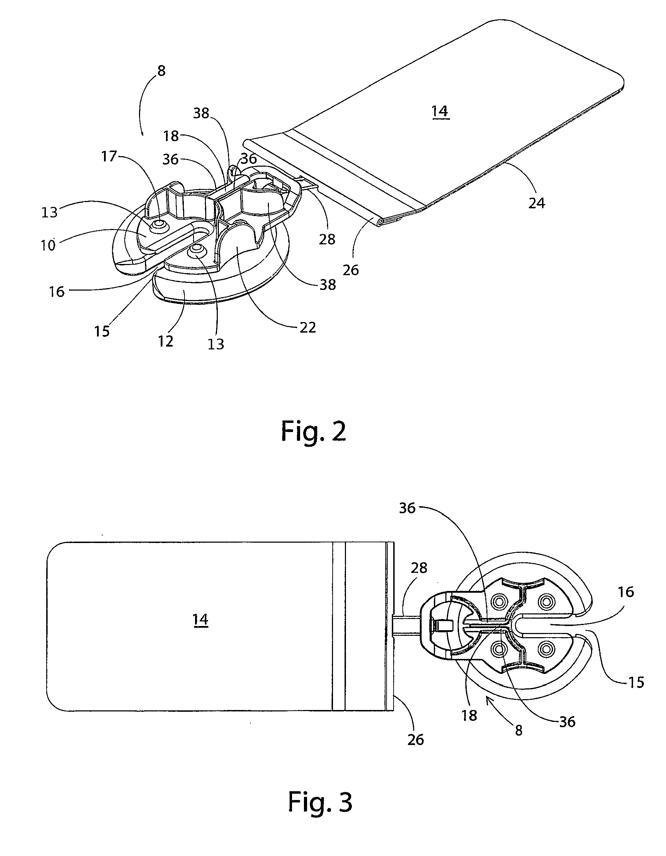

As shown in Figures 2-5, the anchoring portion 10 has a large slot 16 and a smaller slot 18 for engagement of an efac, such as an elastomer. Slot 18 includes walls 36 and is a metered tension, elastomer-locking slot, with a shape, length and size such that the slot 18 captures and anchors the elastomer but allows the elastomer to migrate if tension exceeds a pre-determined level, thereby creating a limit to the amount of force that can be applied by the system. This limit is determined at the time of manufacture of the anchoring portion 10 by controlling the relationship between the size of the slot 18 and the diameter or cross-sectional area of the elastomer. The cross-sectional area of the untensioned portion of the elastomer decreases as the elastomer elongates under increased tension. If a force applied to the elastomer exceeds the therapeutic force range, elongation and resulting reduction in diameter cause the elastomer to release within the slot, returning the quantity of tension to one within the therapeutic limit of the elastomer.

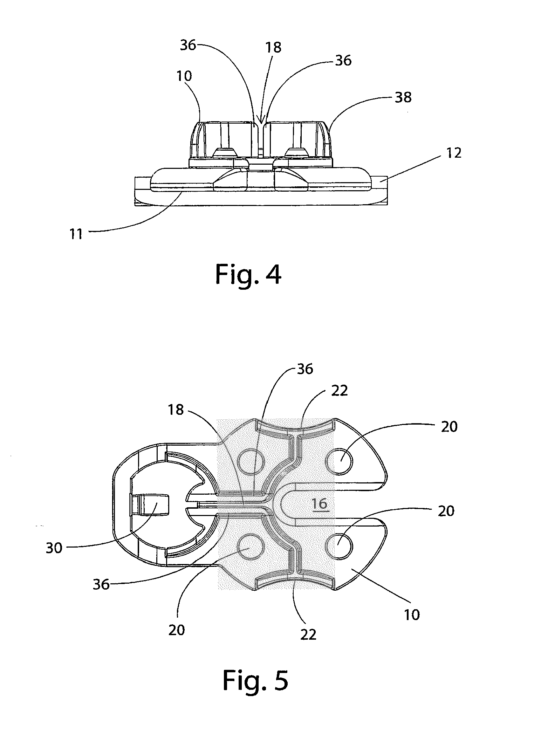

Convex upstanding regions 38 (visible in Figures 1 and 4) of the anchoring portion 10 prevent other objects from catching the edges of the button anchor 8.

The anchoring portion 10 may be molded of polycarbonate plastic or any other appropriately rigid and strong polymeric material suitable for use in the surgical applications for which the present invention is intended. Alternatively it may be molded, machined or otherwise formed or fabricated of any other suitably strong, surgically acceptable material such as stainless steel.

While the size of the button anchor 8 of this invention may be varied depending on the situation in which it is used, anchoring portion 10 may be approximately 32mm in diameter. An anchoring portion 10 for use with an elastomeric three mm diameter, 40 durometer silicone cord may have a slot 18 one mm in width (i.e., the distance between walls 36), 7.3 mm in height and 11mm in length. Many other dimensions are also usable provided that the desired coupling with elastomer is achieved (generally as described above).

Various arcuate or curved surface shapes for anchor efacs attachment structures are described above. It should be understood that functionally equivalent shapes can also be used, such as, for instance, a rod having a cross-section that is not curved but rather is a polygon.

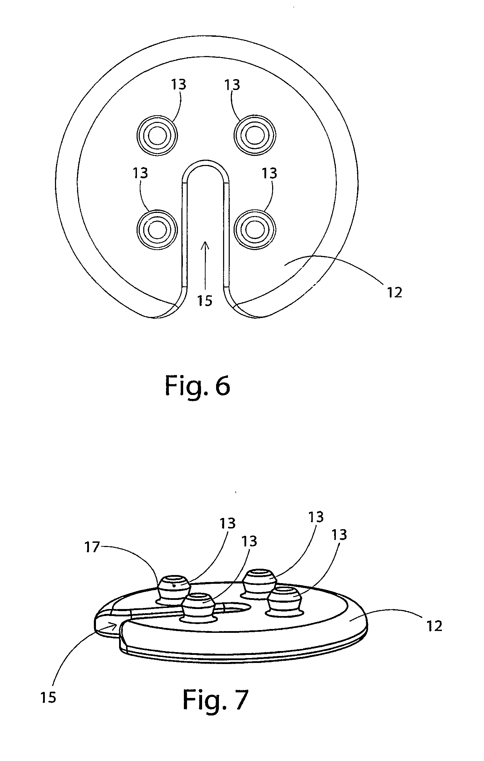

As shown in Figures 6 and 7, anchor pad 12 includes a slot 15 that corresponds to slot 16 of the anchoring portion 10. Anchor pad 12 dissipates the compression load exerted by one or more facs connected to the anchoring portion 10 over the surface of the patient's skin and works to prevent maceration or undue restriction of the underlying blood circulation. The anchor pad 12 is generally the same size and shape as the anchoring portion 10, but it may be smaller or larger in alternative embodiments. For example, larger pads may be used in patients with compromised skin tissues, including the elderly or those with associated co-morbidities, such as diabetes.

The anchor pad 12 may be made of a compressible material such as silicone, or any other suitable material. The skin contact surface (i.e., the underside) of anchor pad 12 may be smooth or it may be textured in order to accommodate fluid dissipation. The skin contact surface may be flat, convex, concave or multi-planar to accommodate anatomical contour. The skin-contacting surface of pad 12 may also be coated or treated to provide antimicrobial properties. In one embodiment, the skin-contacting surface of the anchor pad includes an adhesive.

As shown in Figure 5, the anchoring portion 10 is penetrated by apertures 20 that secure the anchoring portion 10 to the anchor pad 12. Tabs 13 (shown in Figure 7) project from anchor pad 12 and are received in apertures 20 of anchoring portion 10. Enlarged diameter end 17 of tabs 13 retain anchoring portion 10 on pad 12. In an alternative embodiment, the anchor pad 12 is adhered, adhesively bonded, or molded to anchoring portion 10. In one example, the anchor pad 12 and anchoring portion 10 are an integral unit.

As shown in Figures 2 and 5, finger grips 22 facilitate gripping and manipulating the button anchor 8 by opposed digits. Finger grips 22 are concave in the embodiment illustrated in the drawings, but the gripping portion may also be convex, multi-planar or textured.

Optional anchor tail 14, shown in Figures 2, 3 and 8, may be utilized to further dissipate and distribute the shear-load placed on the skin by performing wound closure over the maximum possible surface area. In one embodiment, the anchor tail 14 is formed from polyurethane foam having an adhesive for attachment to the skin and includes a wire that forms a loop 28 at end 26. In alternative embodiments, the anchor tail 14 may be formed from any suitable fabric, foam or film. Such material may be elastic or inelastic. Preferably the anchor tail 14 material conforms to the skin surface and mimics the elasticity of the skin. In addition, the loop 28 may be formed or molded as a separate or integral component.

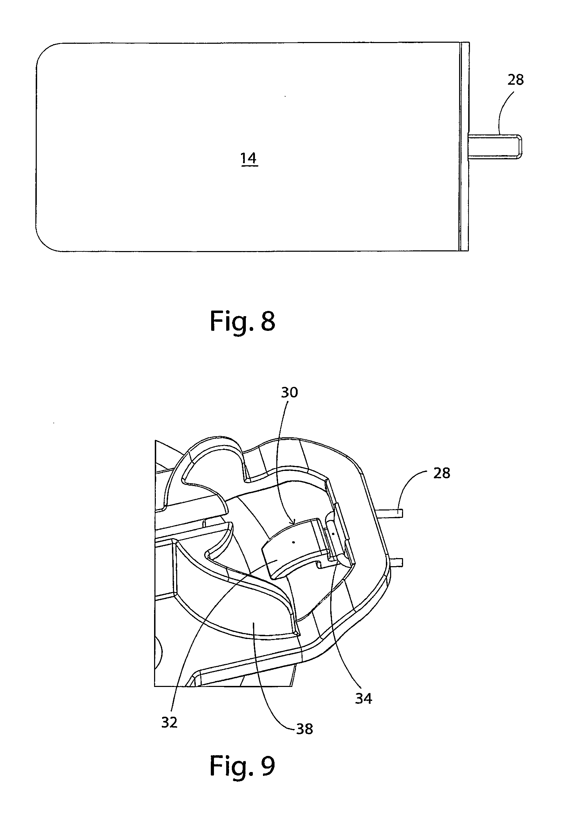

Anchoring portion 10 of button anchor 8 includes structure for engaging anchor tail 14. Such structure may include a hole, tab, cleat or other suitable structure. In one embodiment, shown in the Figures, and particularly in Figure 9, the anchoring portion 10 includes a hook 30 having a ramp 32 for guiding the wire loop 28 of tail 14 up and into depression 34 of anchoring portion 10. In use, the anchor tail 14 is attached to the anchoring portion 10 via the engagement hook 30 and is adhered to the skin. In this manner, anchor tail 14 bolsters the button anchor 8 and dissipates the forward force load (a force vector that travels toward the wound edge and parallel to the skin surface) over a large area of healthy skin located behind the button anchor 8. While the hook 30 and loop 28 provide one example of structure to couple the anchor tail and anchor, any suitable structure may be used.

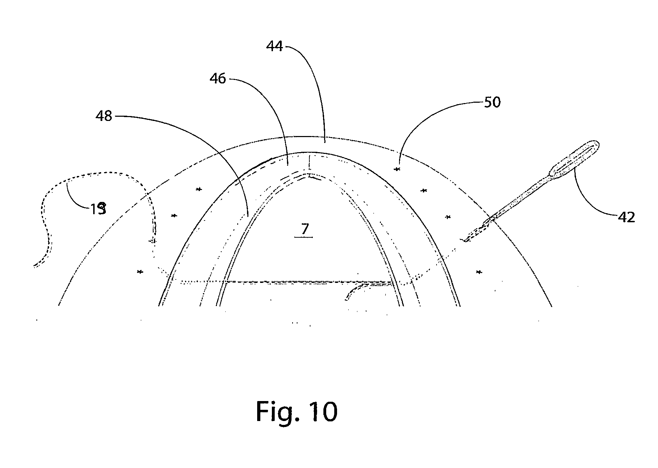

The system of this invention may be used to provide deep fascia repair and deep fascia dynamic wound reduction. In one embodiment, illustrated in Figures 10, a silicone elastomer 19 is coupled to a cannula-like device 42 and is passed through the dermis 44, fat layer 46, and fascia 48 at an optional anchor placement mark 50 placed on the skin prior to installation of the system. After passing through the area of the wound 7, the elastomer 19 is presented through slot 16 of anchoring portion 10 and slot 15 of anchor pad 12 of button anchor 8, where it is then captured and secured in smaller slot 18 of anchoring portion 10. In this manner, closure force is applied to a wound or incision 7. Multiple sets of anchors and elastomers may be used, as shown in Figure 1.

The elastomer 19 may either be presented through the skin and through the slot 16 of an anchor previously placed, or the elastomer 19 may exit the skin, at which time the slot 16 and the pad slot 15 of the anchor 8 may be moved into place around the elastomer 19. The efac may be used to apply tension to sub-dermal structures (deep fascia) but the efac tension may be adjusted from above the skin by increasing or decreasing the tension at the smaller slot 18. The anchor 8 acts as a grommet, removes the point load from the exit hole to reduce the occurrence of localized failures, and also allows adjustment of the tension across the wound. In this manner, the anchor bolsters the perimeter of the transcutaneous opening through which the elastomer passes, reducing localized failures and also reducing scarring.

A system according to this invention may provide wound stabilization of abdominal procedures. For example, this system may be used to restore radial abdominal integrity during prolonged interventions for complications such as abdominal infections management or which require large abdominal access. This system increases patient comfort and mobility by providing abdominal containment and support, and maintains normal skin tensions during intervention to minimize retraction.

Another system of this invention may provide stability to sternal or chest non unions as can arise after open heart surgical procedures. In addition, systems of this invention may be used with conventional primary wound closure methods to distribute skin system tensions to healthy skin beyond the wound, thereby minimizing stress at the wound site and reducing dehiscence. A system of this invention may be applied preoperatively to tension skin and create surplus tissue, allowing excisions to be covered and closed in a conventional manner. Embodiments of this invention may also be used as a dressing retention system by providing efac lacing across the wound site, which passes over the wound dressing and secures it in position. Adhesives may be used on the skin contacting surface of the anchor pad but such adhesives normally would not be required, thereby further facilitating the periodic inspection and cleaning of tissues under the anchor pads.

All of the tissue attachment structure and anchor designs described herein may be produced in a variety of sizes.

The systems of moving or moving and stretching plastic tissue according to this invention are not confined to the embodiments described herein but include variations and modifications within the scope of the foregoing description and the accompanying drawings. For instance, the scale of the components of the invention can vary quite substantially depending on the nature and location of the tissue with which the invention is used. The configuration of the tissue attachment structures can also be varied for the same reasons and for aesthetic reasons. While most of the elements of the illustrative embodiments of the anchors of this invention depicted in the drawings are functional, aspects of the shape and appearance of the illustrative embodiments are nonfunctional and ornamental.

The materials from which the components used in practicing this invention are made can be those described above as well as others, including materials not yet developed that have appropriate properties of strength, elasticity and the like that will be apparent to those skilled in the art in light of the foregoing. For instance, useful materials generally must be sterile or sterilizable and non-reactive. The illustrated components are typically intended to be disposable, but the invention can also be practiced using reusable components.

## Claims

1. A system for moving tissue comprising:
(a) at least one non-reactive elastomeric force applying component (19), and
(b) at least one anchor (10) for attachment to the tissue, the anchor comprising
(i) a first slot (16) sized to allow the elastomeric force applying component to freely pass through the anchor, and
(ii) a second slot (18) sized to capture the elastomeric force applying component without knotting or tearing the elastomeric force applying component and formed to provide adjustable attachment of the elastomeric force applying component, the second slot defined by walls (36); and
(c) at least one anchor pad (12) coupled to the at least one anchor and adapted to distribute force across an area of the tissue,
wherein the anchor distributes force applied to the tissue and bolsters a perimeter of a transcutaneous opening through which the force applying component passes; and wherein **characterized in that** the second slot (18) of the anchor (10) is a metered tension, locking slot, with a shape, length and size such that the slot captures the elastomeric force applying component but allows the component to migrate if tension exceeds a predetermined level and **characterized in that** the pad comprises a pad slot (15) corresponding to the first slot of the anchor allowing the anchor (10) to act as a grommet by removing the point load from the exit hole to reduce the occurrence of localized failures.

2. An anchor assembly for use in a system according to claim 1, for attachment to tissue to transmit force for moving the tissue, the anchor assembly comprising:
an anchor tail (14) comprising adhesive for attachment to the surface of the skin and a loop for engaging the anchor, and
a hook (30) for coupling the anchor tail to the anchor.

3. The anchor assembly of claim 2 wherein the anchor tail (14) further comprises polyurethane foam.

4. The anchor assembly of claim 2 wherein the anchor tail (14) further comprises fabric.

5. The anchor assembly of claim 4 wherein the fabric is elastic and conforms to the skin surface.

6. The anchor assembly of claim 2 wherein the loop (28) comprises wire.

7. The anchor assembly of claim 2, wherein the anchor (10) further comprises a depression (34) and the anchor hook further comprises a ramp for guiding the loop (28) of the anchor tail up and into a depression of the anchor.

8. The system of claim 1 wherein the elastomer comprises silicone.

9. The system of claim 1 wherein the tension is adjustable within an elastic limit of the elastomeric force applying component.

10. The system of claim 1 wherein the elastomeric force applying component is adapted to deform to be released from the anchor upon application of a predetermined force.

11. The system of claim 1 wherein at least two anchors are adapted to be attached to the tissue on opposite sides of a wound or incision.

12. The system of claim 11 wherein the at least two anchors (10) secure at least one elastomeric force applying component and wherein the elastomeric force applying component passes through tissue and fascia.

13. The system of claim 1 or the anchor assembly of claim 2 wherein the anchor pad (12) further comprises a compressible material.

14. The system of claim 1 or the anchor assembly of claim 2 wherein the anchor pad (12) further comprises silicone.

15. The system of claim 1 or the anchor assembly of claim 2, wherein the anchor pad (12) further comprises a skin contacting surface having antimicrobial properties.

16. The system of claim 1 or the anchor assembly of claim 2 wherein the anchor (10) further comprises apertures (20) and the anchor pad (12) further comprises projections that pass through the apertures and couple the anchor pad to the anchor.

17. The system of claim 1 or the anchor assembly of claim 2 wherein the anchor (10) and anchor pad (12) are adhesively bonded.

18. The system of claim 1 or the anchor assembly of claim 2 wherein the anchor (10) and anchor pad (12) are integral.

19. The system of claim 1 or the anchor assembly of claim 2 wherein the anchor (10) further comprises finger grips (22).

20. The system of claim 1 wherein the tissue to be moved is healthy tissue.

## Patentansprüche

1. System zum Bewegen von Gewebe, Folgendes umfassend:
(a) wenigstens eine nichtreaktive elastomere Kraft aufbringende Komponente (19) und
(b) wenigstens einen Anker (10) zum Anbringen am Gewebe, wobei der Anker Folgendes umfasst
(i) einen ersten Schlitz (16), bemessen, um es der elastomeren Kraft aufbringenden Komponente zu ermöglichen, frei durch den Anker hindurch geführt zu werden, und
(ii) einen zweiten Schlitz (18), bemessen, um die elastomere Kraft aufbringende Komponente zu erfassen, ohne die elastomere Kraft aufbringende Komponente zu verknoten oder einzureißen, und geformt, um eine einstellbare Befestigung der elastomeren Kraft aufbringenden Komponente bereitzustellen, wobei der zweite Schlitz durch Wände (36) definiert ist; und
(c) wenigstens eine Ankerunterlage (12), gekoppelt mit wenigstens einem Anker und angepasst, um entlang einem Bereich des Gewebes Kraft zu verteilen,
wobei der Anker auf das Gewebe aufgebrachte Kraft verteilt und einen Umfang einer transkutanen Öffnung abpolstert, durch die die Kraft aufbringende Komponente geführt wird; und wobei der zweite Schlitz (18) des Ankers (10) ein Verriegelungsschlitz mit Spannungsdosierung und von einer solchen Form, Länge und Größe ist, dass der Schlitz die elastomere Kraft aufbringende Komponente erfasst, es der Komponente aber ermöglicht, sich zu bewegen, wenn die Spannung einen vorgegebenen Wert übersteigt, und **gekennzeichnet dadurch, dass** die Unterlage einen Unterlagenschlitz (15) aufweist, der dem ersten Schlitz des Ankers entspricht, und es dem Anker (10) ermöglicht, als Tülle zu dienen, indem die punktförmige Belastung von der Austrittsöffnung abgeleitet wird, um das Auftreten von lokalisierten Schäden zu verringern.

2. Ankeranordnung für den Einsatz in einem System nach Anspruch 1 zum Anbringen an Gewebe, um eine Kraft zu übertragen, um das Gewebe zu bewegen, wobei die Ankeranordnung Folgendes umfasst:
ein Ankerendstück (14), umfassend einen Klebstoff zum Befestigen an der Hautoberfläche und eine Schlaufe für den Eingriff in den Anker
und
einen Haken (30) zum Koppeln des Ankerendstücks an den Anker.

3. Ankeranordnung nach Anspruch 2 wobei das Ankerendstück (14) ferner Polyurethanschaum umfasst.

4. Ankeranordnung nach Anspruch 2, wobei das Ankerendstück (14) ferner Tuch umfasst.

5. Ankeranordnung nach Anspruch 4, wobei das Tuch elastisch ist und sich an die Hautoberfläche anpasst.

6. Ankeranordnung nach Anspruch 2, wobei die Schlaufe (28) Draht umfasst.

7. Ankeranordnung nach Anspruch 2, wobei der Anker (10) ferner eine Vertiefung (34) umfasst und der Ankerhaken ferner eine Schräge umfasst, um die Schlaufe (28) des Ankerendstücks nach oben in eine Vertiefung des Ankers hineinzuführen.

8. System nach Anspruch 1, wobei das Elastomer Silikon umfasst.

9. System nach Anspruch 1, wobei die Spannung innerhalb eines elastischen Grenzwerts der elastomeren Kraft aufbringenden Komponente einstellbar ist.

10. System nach Anspruch 1, wobei die elastomere Kraft aufbringende Komponente angepasst ist, sich zu verformen, um sich beim Aufbringen einer vorgegebenen Kraft vom Anker zu lösen.

11. System nach Anspruch 1, wobei wenigstens zwei Anker angepasst sind, auf einander gegenüberliegenden Seiten einer Wunde oder eines Einschnitts am Gewebe angebracht zu sein.

12. System nach Anspruch 11, wobei die wenigstens zwei Anker (10) wenigstens eine elastomere Kraft aufbringende Komponente sichern und wobei die elastomere Kraft aufbringende Komponente durch Gewebe und Faszien geführt wird.

13. System nach Anspruch 1 oder Ankeranordnung nach Anspruch 2, wobei die Ankerunterlage (12) ferner komprimierbares Material umfasst.

14. System nach Anspruch 1 oder Ankeranordnung nach Anspruch 2, wobei die Ankerunterlage (12) ferner Silikon umfasst.

15. System nach Anspruch 1 oder Ankeranordnung nach Anspruch 2, wobei die Ankerunterlage (12) ferner eine Hautkontaktoberfläche mit antimikrobiellen Eigenschaften aufweist.

16. System nach Anspruch 1 oder Ankeranordnung nach Anspruch 2, wobei der Anker (10) ferner Öffnungen (20) aufweist und die Ankerunterlage (12) ferner Vorsprünge aufweist, die durch die Öffnungen ragen und die Ankerunterlage am Anker befestigen.

17. System nach Anspruch 1 oder Ankeranordnung nach Anspruch 2, wobei der Anker (10) und die Ankerunterlage (12) miteinander verklebt sind.

18. System nach Anspruch 1 oder Ankeranordnung nach Anspruch 2, wobei der Anker (10) und die Ankerunterlage (12) einstückig ausgeführt sind.

19. System nach Anspruch 1 oder Ankeranordnung nach Anspruch 2, wobei der Anker (10) ferner Fingereingriffe (22) aufweist.

20. System nach Anspruch 1, wobei das zu bewegende Gewebe gesundes Gewebe ist.

## Revendications

1. Système de déplacement de tissu comprenant :
(a) au moins un composant d'application de force en élastomère non réactif (19), et
(b) au moins un ancrage (10) pour fixation au tissu, l'ancrage comprenant :
(i) une première fente (16) dimensionnée pour permettre au composant d'application de force en élastomère de passer librement à travers l'ancrage, et
(ii) une seconde fente (18) dimensionnée pour capturer le composant d'application de force en élastomère sans nouer ou déchirer le composant d'application de force en élastomère et formée pour fournir une fixation réglable au composant d'application de force en élastomère, la seconde fente étant définie par des parois (36) ; et
(c) au moins un coussinet d'ancrage (12) couplé à l'au moins un ancrage et adapté pour distribuer une force à travers une superficie du tissu,
dans lequel l'ancrage répartit une force appliquée au tissu et soutient un périmètre d'une ouverture transcutanée à travers laquelle passe le composant d'application de force ; et dans lequel la seconde fente (18) de l'ancrage (10) est une fente de verrouillage à tension mesurée, avec une forme, une longueur et une taille telles que la fente capture le composant d'application de force en élastomère mais permet au composant de migrer si la tension dépasse un niveau prédéterminé et **caractérisé en ce que** le coussinet comprend une fente de coussinet (15) correspondant à la première fente de l'ancrage permettant à l'ancrage (10) de servir de passe-fil, en éliminant la charge ponctuelle du trou de sortie afin de réduire la survenue de défaillances localisées.

2. Ensemble d'ancrage destiné à être utilisé dans un système selon la revendication 1, pour fixation à un tissu afin de transmettre une force permettant de déplacer le tissu, l'ensemble d'ancrage comprenant :
une queue d'ancrage (14) comprenant un adhésif pour fixation à la surface de la peau et une boucle permettant de coopérer avec l'ancrage, et
un crochet (30) permettant de coupler la queue d'ancrage à l'ancrage.

3. Ensemble d'ancrage selon la revendication 2, dans lequel la queue d'ancrage (14) comprend en outre de la mousse polyuréthane.

4. Ensemble d'ancrage selon la revendication 2, dans lequel la queue d'ancrage (14) comprend en outre une étoffe.

5. Ensemble d'ancrage selon la revendication 4, dans lequel l'étoffe est élastique et se conforme à la surface de la peau.

6. Ensemble d'ancrage selon la revendication 2, dans lequel la boucle (28) comprend un fil métallique.

7. Ensemble d'ancrage selon la revendication 2, dans lequel l'ancrage (10) comprend en outre un creux (34) et le crochet d'ancrage comprend en outre une rampe pour guider la boucle (28) de la queue d'ancrage jusque dans un creux de l'ancrage.

8. Système selon la revendication 1, dans lequel l'élastomère comprend du silicone.

9. Système selon la revendication 1, dans lequel la tension est réglable dans une limite élastique du composant d'application de force en élastomère.

10. Système selon la revendication 1, dans lequel le composant d'application de force en élastomère est adapté pour se déformer afin d'être dégagé de l'ancrage lors de l'application d'une force prédéterminée.

11. Système selon la revendication 1, dans lequel au moins deux ancrages sont adaptés pour être fixés au tissu sur des côtés opposés d'une plaie ou d'une incision.

12. Système selon la revendication 11, dans lequel les au moins deux ancrages (10) arriment au moins un composant d'application de force en élastomère et dans lequel le composant d'application de force en élastomère passe à travers le tissu et le fascia.

13. Système selon la revendication 1 ou ensemble d'ancrage selon la revendication 2, dans lequel le coussinet d'ancrage (12) comprend en outre un matériau compressible.

14. Système selon la revendication 1 ou ensemble d'ancrage selon la revendication 2, dans lequel le coussinet d'ancrage (12) comprend en outre du silicone.

15. Système selon la revendication 1 ou ensemble d'ancrage selon la revendication 2, dans lequel le coussinet d'ancrage (12) comprend en outre une surface de contact avec la peau ayant des propriétés antimicrobiennes.

16. Système selon la revendication 1 ou ensemble d'ancrage selon la revendication 2, dans lequel l'ancrage (10) comprend en outre des orifices (20) et le coussinet d'ancrage (12) comprend en outre des protubérances qui passent à travers les orifices et couplent le coussinet d'ancrage à l'ancrage.

17. Système selon la revendication 1 ou ensemble d'ancrage selon la revendication 2, dans lequel l'ancrage (10) et le coussinet d'ancrage (12) sont liés par adhésif.

18. Système selon la revendication 1 ou ensemble d'ancrage selon la revendication 2, dans lequel l'ancrage (10) et le coussinet d'ancrage (12) sont d'un seul tenant.

19. Système selon la revendication 1 ou ensemble d'ancrage selon la revendication 2, dans lequel l'ancrage (10) comprend en outre des brides pour les doigts (22).

20. Système selon la revendication 1, dans lequel le tissu à déplacer est un tissu sain.
